(19) Europäisches Patentamt
European Patent Office
Office européen des brevets

(11) Publication number: 0 099 172
B1

(12) **EUROPEAN PATENT SPECIFICATION**

(45) Date of publication of patent specification: **06.08.86**

(21) Application number: **83302549.7**

(22) Date of filing: **05.05.83**

(51) Int. Cl.⁴: **C 07 D 493/04,**
C 07 D 495/20, C 07 D 491/20
// A61K31/445, A61K31/38,
A61K31/35 ,(C07D493/04,
311:00, 307:00),(C07D495/20,
335:00, 311:00,
307:00),(C07D491/20, 311:00,
307:00, 221:00)

(54) **6,7-Dihydro-7,7-disubstituted-khellins.**

(30) Priority: **17.05.82 US 378701**

(43) Date of publication of application:
**25.01.84 Bulletin 84/04**

(45) Publication of the grant of the patent:
**06.08.86 Bulletin 86/32**

(84) Designated Contracting States:
**BE CH DE FR GB IT LI NL SE**

(56) References cited:
**US-A-4 284 569**
**US-A-4 304 722**
**US-A-4 313 881**

**Chemical Abstracts, vol. 90, no. 23, 4 June 1979, Columbus, Ohio, USA, H. ABU-SHADY et al. "Experiments with khellin. VIII: Synthesis of some 2-3-substituted furochromones", page 653, column 2, abstract no. 186837q**

(73) Proprietor: **THE UPJOHN COMPANY**
**301 Henrietta Street**
**Kalamazoo, Michigan 49001 (US)**

(72) Inventor: **Gammill, Ronald Bruce**
**C/O The Upjohn Company 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 099 172 B1**

**Description**

The present invention relates to novel analogues of a known pharmacological agent, Khellin. Khellin, i.e. 7-methyl-4,9-dimethoxyfurochromone, and related furochromones are naturally-occurring substances and have been used in crude form as pharmacological agents for centuries.

4,9-Dimethoxyfurochromones are known. Such known compounds include 7-ethyl, 7-phenyl, 7-propyl and 7-ethoxycarbonyl analogues described by Schonberg *et al.* JACS *72* (1950) 1611-17; 7-γ-pyridyl analogues, described by Schonberg, JACS *77* (1955) 5439; 7-furanyl analogues described by Musante *et al,* Pharmaco. (Pavie) Ed. Sci. *15* (1960) 81—94; and 7-carboxyaldehyde analogues, described by Mustafa *et al,* J. Org. Chem. *26* (1961) 886. 6-Substituted-4,9-dimethoxyfurochromones are also known; see, for example, Abu-Shady, UAR J. Pharm. Sci. *11* (1970) 283.

4-Methoxy-7-aminomethylenefurochromones are known; see Abu-Shady *et al,* J. Pharm. Belg. *33* (1978) 397.

A wide variety of antiatheroschlereotic furochromones is described in US—A—428569.

Extensive pharmacological uses for khellin and related substances are known. See especially US—A—4284569 and the review by Mustafa, "Furopyrans and Furopyrones", John Wiley and Sons, Inc., N.Y., N.Y. (1967), pp. 102—159 (Chapter III: Furochromones). Also, see US—A—2680119 describing 6- and/ or 7-substituted furochromones, i,e, alkyl, alkoxyalkyl and phenylalkyl substituted compounds.

Novel compounds according to the present invention are of formula I wherein either $R_1$ and $R_2$ are independently selected from:

(a) $C_1$—$C_6$ alkyl,
(b) trifluoromethyl,
(c) $C_5$—$C_{10}$ cycloalkyl, in which there are 5, 6 or 7 C atoms in the ring,
(d) $C_2$—$C_8$ alkylaminoalkyl,
(e) $C_2$—$C_8$ alkoxyalkyl,
(f) $C_2$—$C_8$ alkylthioalkyl,
(g) $C_2$—$C_8$ alkylsulfinylalkyl,
(h) $C_2$—$C_8$ alkylsulfonylalkyl,
(i) $C_7$—$C_{12}$ phenoxyalkyl optionally ring-substituted by one, 2 or 3 substituents selected from hydroxy, $C_1$—$C_3$ alkoxy, $C_1$—$C_3$ alkyl, trifluoromethyl, fluoro, chloro and bromo, with the proviso that not more than two ring-substituents are other than alkyl,
(j) $C_7$—$C_{12}$ phenylthioalkyl optionally ring-substituted as defined above,
(k) phenyl optionally ring-substituted as defined above,
(l) aralkyl optionally ring-substituted as defined above,
(m) 2- or 3-furanyl optionally ring-substituted as defined above,
(n) 2- or 3-thenyl optionally ring-substituted as defined above, and
(o) —$CH_2NR_8R_9$ wherein either $R_8$ and $R_9$ are independently selected from hydrogen, $C_1$—$C_8$ alkyl, $C_5$—$C_{10}$ cycloalkyl, in which there are 5, 6 or 7 C atoms in the ring. $C_7$—C12 aralkyl and phenyl optionally ring-substituted as defined above, or $NR_8R_9$ is a saturated or unsaturated heterocyclic 4- to 8-membered ring consisting of 2 to 7 carbon atoms and zero, one or 2 additional hetero atoms selected from O, N, S, —SO— and —$SO_2$—, in which the heterocyclic ring is optionally substituted by $C_1$—$C_4$ alkyl, $C_2$—$C_8$ alkylthiomethyl, $C_2$—$C_8$ alkoxymethyl, $C_1$—$C_4$ hydroxymethyl or phenyl; or

$R_1$ and $R_2$ are taken together and form a bivalent radical which is:

(a) —$(CH_2)_a$— wherein the integer "a" is from 2 to 7, or
(b) —$(CH_2)_b$—X—$(CH_2)_c$— wherein the integer "b" is one and the integer "c" is one, 2, 3 or 4 or the integer "b" is 2 and the integer "c" is one, 2 or 3, and X is —O—, —S— or —$N(R_{10})$— wherein $R_{10}$ is hydrogen, $C_1$—$C_8$ alkyl, $C_5$—$C_{10}$ cycloalkyl, in which there are 5, 6 or 7 C atoms in the ring. $C_7$—$C_{12}$ aralkyl or phenyl optionally ring-substituted as defined above; and

one of $R_3$ and R is hydrogen or $C_1$—$C_4$ alkoxy and the other is $C_1$—$C_4$ alkoxy.

Preferred compounds of the invention are:

6,7-dihydro-4,9-dimethoxy-7,7-dimethyl-5H-furo[3,2-g][1]benzopyran-5-one,
7-ethyl-6,7-dihydro-4,9-dimethoxy-7-methyl-5H-furo[3,2-g][1]benzopyran-5-one,
7,7-diethyl-6,7-dihydro-4,9-dimethoxy-5H-furo[3,2-g][1]benzopyran-5-one,
6,7-dihydro-4,9-dimethoxy-7-methyl-7-phenyl-5H-furo[3,2-g][1]benzopyran-5-one,
4',9'-dimethoxy-spiro[cyclopentane-1,7'-[7H]furo[3,2-g][1]benzopyran-5'(6'H)-one,
4',9'-dimethoxy-spiro[cyclohexane-1,7'-[7H]furo[3,2-g][1]benzopyran-5'(6'H)-one,
2',3',5',6'-tetrahydro-4,9-dimethoxy-spiro[7H-furo-[3,2-g][1]benzopyran-7,4'-[4H]thiopyran]-5(6H)-one or its 1'-oxide or 1',1'-dioxide,
6,7-dihydro-4,9-dimethoxy-7-methyl-7-[(methylthio)methyl]-5H-furo[3,2-g][1]benzopyran-5-one or its oxide or dioxide, and
4,9-dimethoxy-1'-methyl-spiro[7H-furo[3,2-g][1]benzopyran-7,4'-piperidine]-5(6H)-one.

The carbon atom content of various hydrocarbon containing moieties is indicated by a prefix designating the minimum and maximum number of carbon atoms in moiety, i.e. the prefix $C_i$—$C_j$ indicates a carbon atom content of the integer "i" to the integer "j" carbon atoms, inclusive. Thus, $C_1$—$C_3$ alkyl refers to alkyl of 1—3 carbon atoms, or methyl, ethyl, propyl and isopropyl.

$C_1$—$C_4$ alkyl is methyl, ethyl, propyl or butyl, including isomeric forms thereof. Similarly, $C_1$—$C_6$ alkyl is methyl, ethyl, propyl, butyl, pentyl or hexyl, or an isomeric form thereof.

Examples of $C_5$—$C_{10}$ cycloalkyl are cyclopentyl, cyclohexyl, cycloheptyl, cyclopentylmethyl, cyclohexylmethyl, cycloheptylmethyl, 1- or 2-cyclopentylethyl, 1- or 2-cyclohexylethyl, 1-, 2-, 3- or 4-methylcyclohexyl, (bicyclo[3.1.1]hept-2-yl)methyl, and 6,6-dimethyl-(bicyclo[3.1.1]hept-2-yl)methyl.

Example of alkoxyaminoalkyl are methylaminomethyl, ethylaminomethyl, propylaminomethyl, butylaminomethyl, isopropylaminomethyl, isobutylaminomethyl, tert-butylaminomethyl, pentylaminomethyl, and n-hexylaminomethyl.

Examples of $C_2$—$C_8$ alkoxyalkyl are methoxymethyl, ethoxymethyl, propoxymethyl, butoxymethyl, pentoxymethyl, hexocymethyl, and heptoxymethyl.

Examples of $C_2$—$C_8$ alkylthioalkyl are methylthiomethyl, ethylthiomethyl, propylthiomethyl, butylthiomethyl, isopropylthiomethyl, isobutylthiomethyl, tert-butylthiomethyl, pentylthiomethyl, and n-hexylthiomethyl.

Examples of $C_2$—$C_8$ alkylsulfinylalkyl are methylsulfinylmethyl, ethylsulfinylmethyl, propylsulfinylmethyl, butylsulfinylmethyl, isopropylsulfinylmethyl, isobutylsulfinylmethyl, tert-butylsulfinylmethyl, pentylsulfinylmethyl, and n-hexylsulfinylmethyl.

Examples of $C_2$—$C_8$ alkylsulfonylalkyl are methylsulfonylmethyl, ethylsulfonylmethyl, propylsulfonylmethyl, butylsulfonylmethyl, isopropylsulfonylmethyl, isobutylsulfonylmethyl, tert-butylsulfonylmethyl, pentylsulfonylmethyl, and n-hexylsulfonylmethyl.

Example of $C_6$—$C_{12}$ phenoxyalkyl optionally substituted are phenoxymethyl, phenoxyethyl, phenoxypropyl, phenoxybutyl, phenoxypentyl, 1-, 2-, or 3-methylphenoxymethyl, 1-, 2-, or 3-hydroxyphenoxymethyl, 1-, 2-, or 3-methoxyphenoxymethyl, 1-, 2-, or 3-trifluoromethylphenoxymethyl, 1-, 2-, or 3-fluorophenoxymethyl, and 1-fluoro-3-methyl-phenoxymethyl.

Example of $C_6$—$C_{12}$ phenylthioalkyl optionally substituted are phenylthiomethyl, phenylthioethyl, phenylthiopropyl, phenylthiobutyl, phenylthiopentyl, 1-, 2-, or 3-methylphenylthiomethyl, 1-, 2-, or 3-hydroxyphenylthiomethyl, 1-, 2-, or 3-methoxyphenylthiomethyl, 1-, 2-, or 3-trifluoromethylphenylthiomethyl, 1-, 2-, or 3-fluorophenylthiomethyl, and 1-fluoro-3-methyl-phenylthiomethyl.

Example of $C_6$—$C_{12}$ phenyl optionally substituted are phenyl, 1-, 2-, or 3-methylphenyl, 1-, 2-, or 3-hydroxyphenyl, 1-, 2-, or 3-methoxyphenyl, 1-, 2-, or 3-trifluoromethylphenyl, 1-, 2-, or 3-fluorophenyl, and 1-fluoro-3-methylphenyl.

Examples of aralkyl of 7 to 12 carbon atoms are naphthylmethyl and naphthylethyl.

Examples of heterocyclic amines corresponding to heterocyclic amine rings are thiazolidine, 3-piperidine methanol, 2-piperidine methanol, piperidinic acid, 3-piperidine ethanol, 2-piperidine ethanol, 1-piperizinepropanol, p-piperazinoacetoxyphenone, 4-phenyl-1,2,3,6-tetrahydropyridine, 4-phenylpiperidine, proline, 3-pyrolidinol, tetrahydrofurfurylamine, pyrrolidimethanol, 3-pyrroline, thiazolidine-4-carboxylic acid, thiomorpholine, nipecstamide, morpholine, 2-methylpiperidine, 3-methylpiperidine, 4-methylpiperidine, N-methylpiperazine, and 1-methylhomopiperazine.

Compounds in accordance with the present invention are useful as antiatherosclerotic agents. Thus these compounds are employed by methods known in the art for the use of khellin and related furochromones in the treatment and prevention of atherosclerosis. Accordingly, compounds of formula 1 are employed in humans and in nonhuman mammals at doses from about 0.1—50 mg/kg/day orally. These compounds are used orally in conventional oral dosage forms, including capsules, tablets, and pharmaceutically acceptable liquids. Other routes of administration may also be employed, utilizing equivalent dosages and the appropriate conventional dosage form for the route of administration selected. Such alternatively dosage forms include rectal, vaginal, subcutaneous, intravenous, and like routes of administration.

Compounds of formula I are also useful as food or feed additives whereby the ingestion of food or feed by the mammal being treated results in an effective oral dose of the compound.

Preferred among the compounds of formula I are those wherein at least one or $R_3$ and $R_4$ is methoxy and more preferably both are methoxy.

By virtue of the ring structure of the compounds of the present invention, these substances are named as either 4-alkoxy-, 9-alkoxy-, or 4,9-dialkoxy-, (depending upon whether, respectively, $R_3$ is alkoxy and $R_4$ is hydrogen, $R_4$ is alkoxy and $R_3$ is hydrogen, or $R_3$ and $R_4$ are both alkoxy), 6,7-dihydro-7,7-disubstituted-5H-furo[3,2-g]benzopyran-5-ones when $R_1$ and $R_2$ are not taken together to form a spiro ring. The compounds so described by formula I are 6,7-dihydro-spiro[1,7'-[7H]furo[3,2-g][1]benzopyran)-5'(6'H)-ones.

Novel compounds of the invention are prepared from the known compounds of formula II. The preparation of formula II compunds wherein one or both of $R_3$ and $R_4$ are methoxy is described in US—A—4284569, from khellin or related compounds. Preferably, the formula II compound is prepared by the methods described in EP—A—0094697 or EP—A—0095835.

A formula II compound is reacted with a ketone of formula III, wherein $R_1$, $R_2$, $R_3$ and $R_4$ are as defined above, to give a compound of formula I. This reaction proceeds by combining the reactants with a cyclic amine base, preferably pyrrolidine, and heating for a prolonged period at elevated temperature, e.g. at reflux for from several hours to several days. The product is obtained from the reaction mixture by conventional separation techniques, i.e. filtration, chromatography and crystallisation.

The sulfoxide (—SO—) and sulfone (—$SO_2$—) compounds of formula I are preferably prepared from

corresponding thio (—S—) compounds by oxidisation methods known in the art. A useful oxidising agent for this purpose is m-chloroperoxybenzoic acid and sodium periodate.

The following Examples illustrate the invention.

### Example 1

6,7-Dihydro-4,9-dimethoxy-7,7-dimethyl-5H-furo[3,2-g][1]benzopyran-5-one (I: $R_1 = R_2 = CH_3$, $R_3 = R_4 = OCH_3$)

Toluene (15 ml) and 1-(6-hydroxy-4,7-dimethoxy-5-benzufuranyl)ethanone (6.5 g) are combined with acetone (25 ml) and pyrrolidine (0.75 ml). The resulting mixture is then heated under nitrogen to reflux for 96 hr and then allowed to cool to ambient temperature. Concentration under reduced pressure yields a dark oil which crystallizes. High pressure liquid chromatography on 350 g of silica gel eluting eith ethyl acetate yields 8.4 g of title product as a yellow solid. This solid is diluted with dichloromethane and filtered through basic alumina. The filtrate is then dried and the resulting residue recrystallized from ethyl acetate in hexane to yield 3.8 g of pure title product, melting point 90—92°C. Silica gel TLC $R_f$ is 0.21 in 25% ethyl acetate in hexane. IR absorptions ($cm^{-1}$) are observed at 3140, 1670, 1610, 1595, 1550, 1480, 1355, 1305, 1145, 1080, and 1060. NMR absorptions ($CDCl_3$, δ) are observed at 7.55, 6.95, 4.05, 4.01, 2.72, and 1.50.

### Example 2

7-Ethyl-6,7-dihydro-4,9-dimethoxy-7-methyl-5H-furo[3,2-g][1]benzopyran-5-one (Formula I, $R_1$ is methyl, $R_2$ is ethyl, and $R_3$ and $R_4$ are both methoxy).

Benzene (100 ml) and 1-(6-hydroxy-4,7-dimethoxy-5-benzofuranyl)ethanone (9.68 g) and 2-butanone (10 ml) are combined under a nitrogen atmosphere and pyrrolidine (1 ml) is added. The resulting mixture is then heated to reflux for 72 hr. After cooling to ambient temperature, the reaction mixture is diluted with ethyl acetate and extracted with 2N aqueous sodium hydroxide (50 ml), 2N hdyrochloric acid (100 ml), and brine (20 ml). The organic phase is then dried over magnesium sulphate and filtered. The resulting filtrate is then concentrated under reduced pressure to a residue which is chromatographed on 330 g of silica gel, elluding with 20% ethyl acetate in hexane. Pure title product, 8.0 g of an oil, is obtained. Crystallization of the oil followed by recrystallization yields, 4.04 g of pure title product, melting point 68—71°C. Silica gel TLC $R_f$ is .22 in 25% ethyl acetate in hexane. IR absorptions ($cm^{-1}$) are observed at 3180, 3140, 1685, 1615, 1590, 1550, 1350, 1300, 1140, 1080, and 1060. NMR absorptions ($CDCl_3$, δ) are observed at 7.50, 6.90, 4.04, 4.01, 2.73, 1.82, 1.45, and 1.00.

### Example 3

7,7-Diethyl-6,7-dihydro-4,9-dimethoxy-5H-furo[3,2-g][1]benzopyran-5-one (Formula I, $R_1$ and $R_2$ are both ethyl, and $R_3$ and $R_4$ are both methoxy).

Following the procedure of Example 1 or Example 2, 1-(6-hydroxy-4,7-dimethoxy-5-benzofuranyl)-ethanone (5.1 g) and 3-pentanone (12.8 ml) are transformed to the title product, 2.82 g of crystalline material, melting point 72—73°C. Silica gel TLC $R_f$ is .24 in 25% ethyl acetate in hexane. IR absorptions ($cm^{-1}$) are observed at 3120, 3060, 1670, 1605, 1595, 1500, 1480, 1345, 1315, 1140, 1080, 1065, and 750. NMR absorptions ($CDCl_3$, δ) are observed at 7.50, 6.92, 4.07, 2.75, 1.81, and 0.98.

### Example 4

6,7-Dihydro-4,9-dimethoxy-7-methyl-7-phenyl-5H-furo[3,2-g][1]benzopyran-5-one (Formula I, $R_1$ is methyl, $R_2$ is phenyl, and $R_3$ and $R_4$ are both methoxy).

Following the procedure of Example 1 or Example 2, 1-(6-hydroxy-4,7-dimethoxy-5-benzofuranyl)-ethanone (5.3 g) and acetophenone (6.0 ml) are transformed to 0.5 g of crystalline title product, melting point 161—162°C. Silica gel TLC $R_f$ is .10 in 25% ethyl acetate in hexane. IR absorptions ($cm^{-1}$) are observed at 3130, 1675, 1600, 1550, 1495, 1350, 1315, 1310, 1275, 1140, 1080, 1055, 770, 740, 730, and 705. NMR absorptions ($CDCl_3$, δ) are observed at 7.4, 6.84, 4.15, 3.95, 3.38, 3.04, and 1.80.

### Example 5

4′,9′-Dimethoxy-spiro[cyclopentane-1,7′-[7H]furo[3,2-g][1]benzopyran]-5′(6′H)-one (Formula I, $R_1$ and $R_2$ taken together are $-(CH_2)_4-$, and $R_3$ and $R_4$ are both methoxy).

Following the procedure of Example 1 or Example 2, 1-(6-hydroxy-4,7-dimethoxy-5-benzofuranyl)-ethanone (50 g) and cyclopentanone (29 ml) are transformed to title product, 49.2 g of a yellow solid, melting point 75—77°C. Silica gel TLC $R_f$ is 0.32 in 25% ethyl acetate in hexane. IR absorptions ($cm^{-1}$) are observed at 3120, 3070, 1685, 1610, 1590, 1550, 1480, 1355, 1145, and 1080. NMR absorptions ($CDCl_3$, δ) are observed at 7.55, 6.93, 46.08, 4.00, 2.83, 2.30—1.50.

### Example 6

4′,9′-Dimethoxy-spiro[cyclohexane-1,7′-[7H]furo[3,2-g][1]benzopyran]-5′(6′H)-one (Formula I, $R_1$ and $R_2$ taken together are $-(CH_2)_5-$, and $R_3$ and $R_4$ are both methoxy).

Following the procedure of Example 1 or Example 2, 1-(6-hydroxy-4,7-dimethoxy-5-benzofuranyl)-ethanone (5.97 g) and cyclohexanone (3.45 ml) are transformed to pure title product as a pale yellow crystal (5.93 g), melting point 102—104°C. Silica gel TLC $R_f$ is .30 in 25% ethyl acetate in hexane. IR absorptions

($cm^{-1}$) are observed at 3140, 3120, 1690, 1620, 1540, 1480, 1350, 1280, 1125, and 1060. NMR absorptions ($CDCl_3$, δ) are observed at 7.52, 6.92, 4.08, 2.70, 2.25—1.20.

### Example 7

2',3',5',6'-Tetrahydro-4,9-dimethoxy-spiro[7H-furo[3,2-g][1]benzopyran-7,4'-(4H)thiopyran]-5(6H)-one (Formula I, $R_1$ and $R_2$ taken together are $—(CH_2)_2—S—(CH_2)_2—$, and $R_3$ and $R_4$ are both methoxy)..

Following the procedure of Example 1 or Example 2, 1-(6-hydroxy-4,7-dimethoxy-5-benzofuranyl)-ethanone (10.63 g) and tetrahydrothiopyran-4-one (7.8 g) are transformed to pure title product (9.42 g), melting point 146.7—147.9°C. Silica gel TLC $R_f$ is .1 in 25% ethyl acetate in hexane. IR absorptions ($cm^{-1}$) are observed at 3140, 3120, 1685, 1620, 1535, 1475, 1350, 1125, and 1065. NMR absorptions ($CDCl_3$, δ) are observed at 7.55, 6.95, 4.10, 4.07, 3.45, 2.80, 2.72, 2.7—1.5.

### Example 8

2',3',5',6'-Tetrahydro-4,9-dimethoxy-spiro[7H-furo(3,2-g][1]benzopyran-7,4'£[4H]thiopyran]-5(6H)-one, 1'-oxide and 2', 3', 5', 6'-Tetrahydro-4,9-dimethoxy-spiro(7H-furo[3,2-g][1]benzopyran-7,4'-(4H]thiopyran]-5(6H)-one, 1',1'-dioxide (Formula I: $R_1$ and $R_2$ taken together are $—(CH_2)_2—SO—(CH_2)_2—$ or $—(CH_2)_2—SO_2—(CH_2)_2—$, respectively, and R and $R_4$ are both methoxy.

A. A solution of the title product of Example 7 [4.15 g) in a mixture of methanol and tetrahydrofuran (1:1) are diluted with water (75 ml) and then treated with sodium periodate (2.7 g). After about 30 min a precipitate appears. The reaction is then continued with stirring at ambient temperature for 12 hr and filtered. The filter cake is then washed with methanol (100 ml) and the total filtrate concentrated under reduced pressure to a residue, a mixture of title product.

B. The mixture of Part A is then combined with dichloromethane and m-chloroperoxybenzoic acid (2.1 g) and stirred for 16 hrs. Treatment with 2 normal sodium hydroxide (75 ml) is followed by stirring for 5 min and separation of phases. The organic layer is then dried over sodium sulfate and concentrated under reduced pressure to a residue. Chromatography on silica gel eluting with ethyl acetate in trichloromethane (1:1) yields 4 g of sulfone. Recrystallization from acetone and hexane (5:1) yields 3.6 g of pure title product. Silica gel TLC $R_f$ is 0.33 in ethyl acetate. IR absorptions ($cm^{-1}$) are observed at 3160, 3120, 3080, 1685, 1605, 1595, 1555, 1475, 1315, 1295, 1135, 1100, 1080, and 1070. NMR absorptions ($CDCl_3$, δ) are observed at 7.61, 6.99, 4.18, 4.13, 3.8—2.9, 2.78, and 2.75—2.2.

### Example 9

6,7-Dihydro-4,9-dimethoxy-7-methyl-7-[(methylthio)methyl]-5H-furo[3,2-g][1]benzopyran-5-one (Formula I, $R_1$ is methyl, $R_2$ is methylthiomethyl, and $R_3$ and $R_4$ are both methoxy).

Following the procedure of Example 1 or Example 2, 1-(6-hydroxy-4,7-dimethoxy-5-benzofuranyl)-ethanone (8.33 g) and thiomethyl acetone (5.5 ml) are transformed to 7.5 g of crystalline title product. Recrystallization from methyl acetate in hexane yields pure crystalline product, melting point 64—66°C. Silica gel TLC $R_f$ is .40 in 50% ethyl acetate in hexane. IR absorptions ($cm^{-1}$) are observed at 3160, 3120,. 3060, 1680, 1615, 1590, 1545, 1475, 1305, 1160, 1150, 1140, 1080, and 1065. NMR absorptions ($CDCl_3$, δ) are observed at 7.55, 6.95, 4.05, 4.01, 3.16, 2.93, 2.60, 2.26, and 1.58.

### Example 10

6,7-Dihydro-4,9-dimethoxy-7-methyl-7-[(methylthio)methyl]-5H-furo[3,2-g][1]benzopyran-5-one oxide and 6,7-Dihydro-4,9-dimethoxy-7-methyl-7-[(methylthio)methyl]-5H-furo[3,2-g][1]benzopyran-5-one, dioxide (Formula I: $R_1$ is methyl, $R_2$ is methylsulfinylmethyl or methylsulfonylmethyl, respectively, and $R_3$ and $R_4$ are both methoxy).

Following the procedure of Example 8, the product of Example 7 is converted to a corresponding sulfoxide or sulfone.

### Example 11

4,9-Dimethoxy-1'-methyl-spiro[7H-furo[3,2-g][1]benzopyran-7,4'-piperidine)-5(6H)-one (Formula I, $R_1$ and $R_2$ taken together are $—(CH_2)_2—N(CH_3)—(CH_2)_2—$, and $R_3$ and $R_4$ are both methoxy).

Following the procedure of Example 1 or Example 2,, 1-(6)hydroxy-4,7-dimethoxy-5-benzofuranyl)-ethanone (7.35 g) and N-methyl-4-piperidone (6 ml) are transformed to 6.72 g of title product as a tan crystal, melting point 102—103.1°C. Silica gel TLC $R_f$ is .21 in 10% methanol in trichloromethane. IR absorptions ($cm^{-1}$) are observed at 3100, 3060, 2800, 2780, 2760, 1685, 1760, 1625, 1610, 1590, 1545, 1540, 1480, 1150, 1130, and 1075. NMR absorptions ($CDCl_3$, δ) are observed at 7.51, 6.94, 4.10, 4.07. 2.72, 2.7—2.43, 2.35, and 2.2—1.6.

In accordance with the procedures of the examples given above, there are prepared each of the various novel Formula I compounds disclosed herein.

FORMULAS

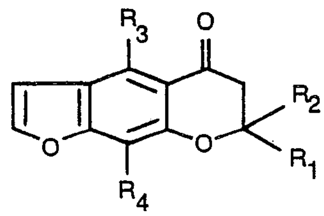

I

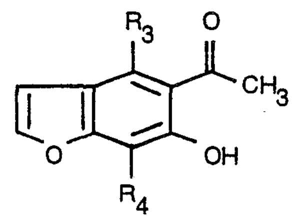

II

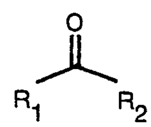

III

**Claims**

1. A dihydrofurochromone of formula I

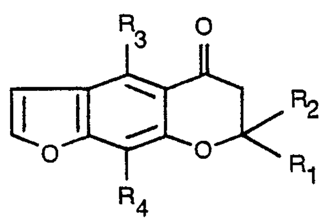

I

wherein either $R_1$ and $R_2$ are independently selected from:

(a) $C_1$—$C_6$ alkyl,

(b) trifluoromethyl,

(c) $C_5$—$C_{10}$ cycloalkyl, in which there are 5, 6 or 7 C atoms in the ring,

(d) $C_2$—$C_8$ alkylaminoalkyl,

(e) $C_2$—$C_8$ alkoxyalkyl,

(f) $C_2$—$C_8$ alkylthioalkyl,

(g) $C_2$—$C_8$ alkylsulfinylalkyl,

(h) $C_2$—$C_8$ alkylsulfonylalkyl,

(i) $C_7$—$C_{12}$ phenoxyalkyl optionally ring-substituted by one, 2 or 3 substituents selected from hydroxy, $C_1$—$C_3$ alkoxy, $C_1$—$C_3$ alkyl, trifluoromethyl, fluoro, chloro and bromo, with the proviso that not more than two ring-substituents are other than alkyl,

(j) $C_7$—$C_{12}$ phenylthioalkyl optionaly ring-substituted as defined above,

(k) phenyl optionally ring-substituted as defined above,

(l) aralkyl optionally ring-substituted as defined above,

(m) 2- or 3-furanyl optionally ring-substituted as defined above,

(n) 2- or 3-thenyl optionally ring-substituted as defined above, and

(o) —$CH_2NR_8R_9$ wherein either $R_8$ and $R_9$ are independently selected from hydrogen, $C_1$—$C_8$ alkyl, $C_5$—$C_{10}$ cycloalkyl, in which there are 5, 6 or 7 C atoms in the ring. $C_7$—$C_{12}$ aralkyl and phenyl optionally ring-substituted as defined above, or $NR_8R_9$ is a saturated or unsaturated heterocyclic 4- to 8-membered ring consisting of 2 to 7 carbon atoms and zero, one or 2 additional hetero atoms selected from O, N, S, —SO— and —$SO_2$—, in which the heterocyclic ring is optionally substituted by $C_1$—$C_4$ alkyl, $C_2$—$C_8$ alkylthiomethyl, $C_2$—$C_8$ alkoxymethyl, $C_1$—$C_4$ hydroxymethyl or phenyl; or

$R_1$ and $R_2$ are taken together and form a bivalent radical which is:

(a) —$(CH_2)_a$— wherein the integer "a" is from 2 to 7, or

(b) —$(CH_2)_b$—X—$(CH_2)_c$— wherein the integer "b" is one and the integer "c" is one, 2, 3 or 4 or the integer "b" is 2 and the integer "c" is one, 2 or 3, and X is —O—, —S— or —$N(R_{10})$— wherein $R_{10}$ is

hydrogen, $C_1$—$C_8$ alkyl, $C_5$—$C_{10}$ cycloalkyl, in which there are 5, 6 or 7 C atoms in the ring. $C_7$—$C_{12}$ aralkyl or phenyl optionally ring-substituted as defined above; and

one of $R_3$ and $R_4$ is hydrogen or $C_1$—$C_4$ alkoxy and the other is $C_1$—$C_4$ alkoxy.

2. A compound as claimed in claim 1, wherein at least one of $R_3$ and $R_4$ is methoxy.

3. A compound as claimed in claim 2, wherein $R_3$ and $R_4$ are each methoxy.

4. A compound selected from
6,7-dihydro-4,9-dimethoxy-7,7-dimethyl-5H-furo[3,2-g][1]benzopyran-5-one,
7-ethyl-6,7-dihydro-4,9-dimethoxy-7-methyl-5H-furo[3,2-g][1]benzopyran-5-one,
7,7-diethyl-6,7-dihydro-4,9-dimethoxy-5H-furo[3,2-g][1]benzopyran-5-one, and
6,7-dihydro-4,9-dimethoxy-7-methyl-7-phenyl-5H-furo[3,2-g][1]benzopyran-5-one.

5. 4',9'-dimethoxy-spiro[cyclopentane-1,7'-[7H]furo[3,2-g][1]benzopyran-5'(6'H)-one, or
4',9'-dimethoxy-spiro[cyclohexane-1,7'-[7H]furo[3,2-g][1]benzopyran-5'(6'H)-one.

6. 2',3',5',6'-tetrahydro-4,9-dimethoxy-sprio[7H-furo-[3,2-g][1]benzopyran-7,4'-[4H]thiopyran]-5(6H)-one or its 1'-oxide or 1',1'-dioxide.

7. 6,7-dihydro-4,9-dimethoxy-7-methyl-7-[(methylthio)methyl]-5H-furo[3,2-g][1]benzopyran-5-one or its oxide or dioxide.

8. 4,9-dimethoxy-1'-methyl-spiro[7H-furo[3,2-g][1]benzopyran-7,4'-piperidine]-5(6H)-one.

**Patentansprüche**

1. Ein Dihydrofurochromon der Formel I

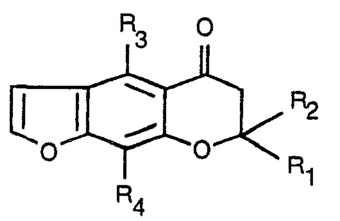

I

wobei $R_1$ und $R_2$ unanhängig gewählt werden von:

(a) $C_1$—$C_6$ Alkyl,
(b) Trifluoromethyl,
(c) $C_5$—$C_{10}$ Cycloalkyl, mit 5,6 oder 7 C-Atomen im Ring,
(d) $C_2$—$C_8$ Alkylaminoalkyl,
(e) $C_2$—$C_8$ Alkoxyalkyl,
(f) $C_2$—$C_8$ Alkylthioalkyl,
(g) $C_2$—$C_8$ Alkylsulfinylalkyl,
(h) $C_2$—$C_8$ Alkylsulfonylalkyl,
(i) $C_7$—$C_{12}$ Phenoxyalkyl wahlweise ringsubstituiert durch ein, 2 oder 3 Substituenten, gewählt von Hydroxy, $C_1$—$C_3$ Alkoxy, $C_1$—$C_3$ Alkyl, Trifluoromethyl, Fluoro, Chloro und Bromo, unter der Voraussetzung, daß nicht mehr als zwei Ring-Substituenten etwas anderes sind als Alkyl,
(j) $C_7$—$C_{12}$ Phenylthioalkyl wahlweise ringsubstituiert wie oben definiert,
(k) Phenyl wahlweise ringsubstituiert wie oben definiert,
(l) Aralkyl wahlwise ringsubstituiert wie oben definiert,
(m) 2- oder 3-Furanyl wahlweise ringsubstituiert wie oben definiert,
(n) 2- oder 3-Thenyl wahlweise ringsubstituiert wie oben definiert, und
(o) —$CH_2NR_8R_9$ wobei jedes $R_8$ und $R_9$ unabhängig gewählt wird aus Wasserstoff, $C_1$—$C_8$ Alkyl, $C_5$—$C_{16}$ Cycloalkyl mit 5,6 oder 7 C-Atomen im Ring, $C_7$—$C_{12}$ Aralkyl und Phenyl wahlwise ringsubstituiert wie oben definiert, oder $NR_8R_9$ ist ein gesättigter oder ungesättigter heterocyclischen 4- bis 8-gliedriger Ring bestehend aus 2 bis 7 Kohlestoffatomen und null, ein oder 2 zusätzlichen Heteroatomen gewählt aus O, N, S, —SO— und —$SO_2$—, in denen der heterocyclische Ring wahlweise substituiert wird durch $C_1$—$C_4$ Hydroxymethyl oder Phenyl; oder

$R_1$ und $R_2$ werden zusammengenommen und bilden ein bivalentes Radikal, nämlich:
(a) —$(CH_2)_a$— wobei die Ganzzahl "a" 2 bis 7 ist oder
(b) —$(CH2)_b$—X—$(CH_2)_c$— wobei die Ganzzahl "b" eins und die Ganzzahl "c" eins, 2,3 oder 4 ist oder die Ganzzahl "b" ist 2 und die Ganzzahl "c" ist eins, 2 oder 3, und X ist —O—, —S— oder —$N(R_{10})$— wobei $R_{10}$ Wasserstoff, $C_1$—$C_8$ Alkyl, $C_5$—$C_{10}$ Cycloalkyl mit 5,6 oder 7 C-Atomen im Ring, $C_7$—$C_{12}$ Aralkyl oder Phenyl wahlweise ringsubstituiert wie oben definiert ist; und

ein $R_3$ und $R_4$ ist Wasserstoff oder $C_1$—$C_4$ Alkoxy und das andere $C_1$—$C_4$ Alkoxy.

2. Eine Verbindung wie in Anspruch 1, dargestellt, wobei mindestens ein $R_3$ und $R_4$ Methoxy ist.

3. Eine Verbindung wie in Anspruch 2, dargestellt, wobei $R_3$ und $R_4$ beide Methoxy sind.

4. Eine Verbindung gewählt aus
6,7-Dihydro-4,9-Dimethoxy-7,7-Dimethyl-5H-Furo[3,2-g][1]Benzopyran-5-on,

7-Äthyl-6,7-Dihydro-4,9-Dimethoxy-7-Methyl-5H-Furo[3,2-g][1]Benzopyran-5-on,
7,7-Diäthyl-6,7-Dihydro-4,9-Dimethoxy-5H-Furo[3,2-g][1]Benzopyran-5-on, und
6,7-Dihydro-4,9-Dimethoxy-7-Methyl-8-Phenyl-5H-Furo[3,2-g][1]Benzopyran-5-on.

5. 4',9'-Dimethoxy-Spiro[Cyclopentane-1,7'-[7H]Furo[3,2-g][1]Benzopyran-5'(6'H)-on, oder
4',9'-Dimethoxy-Spiro[Cyclohexan-1,7'-[7H]Furo[3,2-g][1]Benzopyran-5'(6'H)-on.

6. 2',3',5',6'-Tetrahydro-4,9-Dimethoxy-Spiro[7H-Furo-[3,2-g][1]Benzopyran-7,4'-[4H]Thiopyran]-5(6H)-on oder sein 1'-Oxid oder 1',1'-Dioxid.

7. 6,7-Dihydro-4,9-Dimethoxy-7-Methyl-7-[(Methylthio)Methyl]-5H-Furo[3,2-g][1]Benzopyran-5-on oder sein Oxid or Dioxid.

8. 4,9-Dimethoxy-1'-Methyl-Spiro[7H-Furo[3,2-g][1]Benzopyran-7,4'-Piperidin]-5(6H)-on.

**Revendications**

1. Dihydrofurchromone de formule I

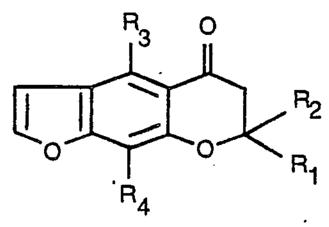

I

dans laquelle $R_1$ et $R_2$ sont choisis, indépendamment, entre:

(a) un groupe alkyle en $C_1$ à $C_6$,
(b) le groupe trifluorométhyle,
(c) un groupe cycloalkyle en $C_5$ à $C_{10}$ dont is noyau comprend 5, 6 ou 7 atomes de carbone,
(d) un groupe alkylaminoalkyle en $C_2$ à $C_8$,
(e) un groupe alkoxyalkyle en $C_2$ à $C_8$,
(f) un groupe alkylthioalkyle en $C_2$ à $C_8$,
(g) un groupe alkylsulfinylalkyle en $C_2$ à $C_8$,
(h) un groupe alkylsulfonylalkyle en $C_2$ à $C_8$,
(i) un groupe phénoxyalkyle en $C_7$ à $C_{12}$ éventuellement substitué sur le noyau par 1, 2 ou 3 substituants choisis entre des substituants hydroxy, alkoxy en $C_1$ à $C_3$, alkyle en $C_1$ à $C_3$, trifluorométhyle, fluoro, chloro et bromo, sous réserve que pas plus de deux substituants du noyau représentent autre chose que des groupes alkyle,
(j) un groupe phénylthioalkyle en $C_7$ à $C_{12}$ éventuellement substitué sur le noyau comme défini ci-dessus,
(k) un groupe phényle éventuellement substitué sur le noyau comme défini ci-dessus,
(l) un groupe aralkyle éventuellement substitué sur le noyau comme défini ci-dessus,
(m) un groupe 2- ou 3-furannyle éventuellement substitué sur le noyau comme défini ci-dessus,
(n) un groupe 2- ou 3-thényle éventuellement substitué sur le noyau comme défini ci-dessus, et
(o) un groupe $-CH_2NR_8R_9$ dans lequel $R_8$ et $R_9$ sont choisis indépendamment entre l'hydrogène, un groupe alkyle en $C_1$ à $C_8$, cycloalkyle en $C_5$ à $C_{10}$ dont le noyau comprend 5, 6 ou 7 atomes de carbone, aralkyle en $C_7$ à $C_{12}$ et phényle éventuellement substitué sur le noyau comme défini ci-dessus, ou bien $NR_8R_9$ est un noyau hétérocyclique saturé ou insaturé de 4 à 8 chaînons comprenant 2 à 7 atomes de carbone et zéro, un ou 2 autres hétéroatomes choisis entre O, N, S, $-SO-$ et $-SO_2-$, le noyau hétérocyclique étant éventuellement substitué par un substituant alkyle en $C_1$ à $C_4$, alkylthiométhyle en $C_2$ à $C_8$, alkoxyméthyle en $C_2$ à $C_8$, hydroxyméthyle en $C_1$ à $C_4$ ou phényle; ou bien

$R_1$ et $R_2$, considérés ensemble, forment un radical bivalent qui est:
(a) un radical $-(CH_2)_a-$ dans lequel le nombre entier "a" a une valeur de 2 à 7, ou bien
(b) un radical $-(CH_2)_b-X-(CH_2)_c-$ dans lequel le nombre entier "b" est égal à un et le nombre entier "c" est égal à un, 2, 3 ou 4, ou bien le nombre entier "b" est égal à 2 et le nombre entier "c" est égal à un, 2 ou 3, et X représente $-O-$, $-S-$ ou $-N(R_{10})-$ où $R_{10}$ est l'hydrogène, un groupe alkyle en $C_1$ à $C_8$, cycloalkyle en $C_5$ à $C_{10}$ dont le noyau comprend 5, 6 ou 7 atomes de carbone, un groupe aralkyle en $C_7$ à $C_{12}$ ou phényle éventuellement substitué sur le noyau comme défini ci-dessus; et

l'un de $R_3$ et $R_4$ représente l'hydrogène ou un groupe alkoxy en $C_1$ à $C_4$ et l'autre est un groupe alkoxy en $C_1$ à $C_4$.

2. Composé suivant la revendication 1, dans lequel au moins un de $R_3$ et $R_4$ est un groupe méthoxy.

3. Composé suivant la revendication 2, dans lequel $R_3$ et $R_4$ sont chacun un groupe méthoxy.

4. Composé choisi entre
la 6,7-dihydro-4,9-diméthoxy-7,7-diméthyl-5H-furo[3,2-g][1]benzopyranne-5-one,

la 7-éthyl-6,7-dihydro-4,9-diméthoxy-7-méthyl-5H-furo[3,2-g][1]benzopyranne-5-one,
la 7,7-diéthyl-6,7-dihydro-4,9-diméthoxy-5H-furo[3,2-g][1]benzopyranne-5-one,
la 6,7-dihydro-4,9-diméthoxy-7-méthyl-7-phényl-5H-furo[3,2-g][1]benzopyranne-5-one.

5. La 4',9'-diméthoxy-spiro[cyclopentane-1,7'-[7H]furo[3,2-g][1]benzopyranne-5'(6'H)-one ou la 4',9'-diméthoxy-spiro[cyclohexane-1,7'-[7H]furo[3,2-g][1]benzopyranne-5'(6'H)-one.

6. La 2',3',5',6'-tétrahydro-4,9-diméthoxy-spiro[7H-furo-[3,2-g][1]benzopyranne-7,4'-[4H]thiopyranne]-5(6H)-one ou son 1'-oxyde ou son 1',1'-dioxyde.

7. La 6,7-dihydro-4,9-diméthoxy-7-méthyl-7-[(méthylthio)méthyl]-5H-furo[3,2-g][1]benzopyranne-5-one ou son oxyde ou son dioxyde.

8. La 4,9-diméthoxy-1'-méthyl-spiro[7H-furo[3,2-g][1]benzopyranne-7,4'-pipéridine]-5(6H)-one.